# EUROPÄISCHE PATENTSCHRIFT

(11) **EP 0 287 876 B1**
(45) Veröffentlichungstag und Bekanntmachung des Hinweises auf die Patenterteilung: **27.01.1993**
(21) Anmeldenummer: 88105274.0
(22) Anmeldetag: 31.03.1988
(51) Int. Cl.: A61K 7/06, A61K 7/075, A61K 7/08

(54) **Haarregenerierende Zubereitungen**
Hair-regenerating compositions
Compositions régénérant les cheveux

(30) Priorität: 08.04.1987 DE 3711841
(43) Veröffentlichungstag der Anmeldung: 26.10.1988
(73) Patentinhaber: Henkel Kommanditgesellschaft auf Aktien, 40191 Düsseldorf (DE)
(72) Erfinder: Wisotzki, Klaus-Dieter, Dr., D-4006 Erkrath 2 (DE); Höffkes, Horst, Dr., D-4000 Düsseldorf (DE); Hollenberg, Detlef, Dr., D-4010 Hilden (DE)

(56) Entgegenhaltungen:
- EP-A- 0 005 807
- EP-A- 0 102 736
- US-A- 4 656 043
- DRUG & COSMETIC INDUSTRY, Band 116, Nr. 6, Juni 1975; W.R.DRISCOLL, Seiten 42-45, 149-153

## Beschreibung

Die Erfindung betrifft Zubereitungen zur Haarbehandlung, die eine Wirkstoffkombination zur Pflege und Gesundung strapazierter Haare, insbesondere zur Regenerierung durch Haarspliss geschädigter Haare enthält.

Haarspliss ist eine Erscheinung, die sich in einem Poröswerden des Haarschaftes und einer Aufspaltung des Haars von der Haarspitze her manifestiert. Haarspliss wird u. a. durch starke mechanische Beanspruchung des Haars, z.B. durch häufiges Bürsten, Toupieren oder Kämmen gegen hohen Kämmwiderstand verursacht. Ursache für hohen Kämmwiderstand bei trockenem Haar kann Schädigung der Haaroberfläche, statische Aufladung oder Klebrigkeit infolge von Rückständen von Haarsprays sein. Die Gefahr von Haarspliss wird auch durch eine Schwächung der Haarstruktur erhöht, die durch häufige oder unsachgemäße Anwendung chemischer Behandlungsverfahren, z.B. beim Dauerwellen oder Färben, verursacht sein kann.

Es hat daher nicht an Versuchen gefehlt, durch Haarspliss geschädigtes Haar mit Hilfe geeigneter Zubereitungen zu regenerieren, das heißt, den weiteren Fortschritt des Haarspliss aufzuhalten und das gesplisste Haar zu sanieren.

Panthenol (2,4-Dihydroxy-N-(3-hydroxypropyl)-3,3-dimethylbutyramid) ist als Bestandteil konditionierender Haarbehandlungsmittel bekannt (vgl. Drug Cosmetic Ind. Juni 1975, 42-45, 149-153).

Auch reduzierend wirkende Zucker sind bereits als strukturverbessernde Zusätz zu Haarbehandlungsmitteln beschrieben worden (z.B. in DE-A-28 24 025).

Die bekannten Mittel zeigen aber nur dann eine nennenswerte Wirkung, wenn sie nach der Anwendung auf dem Haar verbleiben, wie dies z.B. bei Haarfestigern oder Haarwässern der Fall ist. Hingegen ist es bisher nicht gelungen, aus abspülbaren Haarbehandlungsmitteln, wie z.B. Haarkuren, Haarspülmitteln oder gar Shampoos eine befriedigende haarregenerierende Wirkung zu erzielen, da bei diesen Produkten die Einwirkungszeit relativ kurz ist.

Es wurde nun gefunden, daß durch eine Kombination von Panthenol und einem Mono- oder Disaccharid die haarregenerierende Wirkung so stark gesteigert werden kann, daß schon nach sehr kurzer Einwirkungsdauer eine hohe "Heilungsrate" bei stark gesplisstem Haar beobachtet werden kann.

Gegenstand der Erfindung sind daher Haarbehandlungsmittel in Form wässriger, wässrig-alkoholischer oder emulsionsförmiger Zubereitungen zur Pflege strapazierter Haare, die dadurch gekennzeichnet sind, daß sie
0,1 - 8 Gew.-% Panthenol und
0,1 - 8 Gew.-% eines Mono- oder Disaccharids
enthalten.

Das Panthenol kann sowohl in der D-Form als auch als Racemat (D, L-Form) vorliegen. Es hat sich aber gezeigt, daß das D-(+)-Panthenol eine höhere Wirksamkeit aufweist und daher bevorzugt zur Anwendung kommt.

Als Mono- oder Disaccharid kommen alle Aldosen und Ketosen, insbesondere die Pentosen (5 C-Atome) und Hexosen (6 C-Atome) sowie die aus Pentosen und Hexosen zugänglichen Disaccharide infrage. Geeignete Monosaccharide sind z.B. Glucose, Mannose, Galactose, Ribose, Arabinose, Xylose, Fructose und Sorbose, geeignete Disaccharide sind z.B. Saccharose (Rohrzucker), Lactose (Milchzucker), Maltose (Malzzucker) und Cellobiose. Geeignet sind auch natürlich vorkommende oder technische Gemische, in welchen die genannten Mono- oder Disaccharide überwiegend enthalten sind, z.B. Honig, Zuckersirup, Invertzuckerlösungen. Bevorzugt eingesetzt wird Glucose, die sowohl in der D-(+)- oder L-(-)-Konfiguration oder als Racemat geeignet ist.

Weiterhin wurde gefunden, daß die Wirksamkeit der Kombination aus Panthenol und dem Mono- oder Disaccharid weiter gesteigert werden kann, wenn die erfindungsgemäßen Haarbehandlungsmittel zusätzlich
0,1 - 4 Gew.-% eines Polyvinylpyrrolidons und/oder
0,01 - 1 Gew.-% eines Triols der allgemeinen Formel I 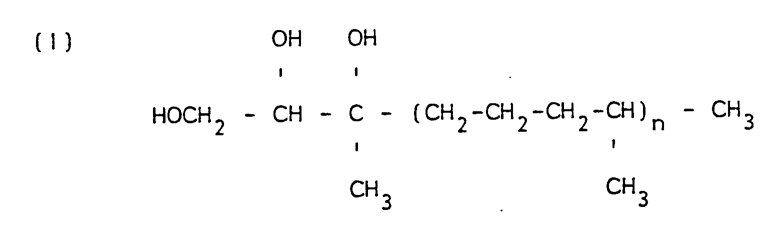
in der n = 1-3 ist, enthält.

Polyvinylpyrrolidon (PVP) ist als filmbildender Bestandteil von Haarpflegemitteln seit langem bekannt. Die handelsüblichen Produkte können ein mittleres Molekulargewicht von ca. 10 000 bis 360 000 (K-Wert 15 bis 100) aufweisen, sind gut wasserlöslich und innerhalb des genannten mittleren Molekulargewichtsbereiches bereits praktisch alle gleichermaßen zur Ausführung der Erfindung geeignet.

Triole der allgemeinen Formel I sind aus DE-A-1 149 700 bekannt. Unter den aus dieser Druckschrift bekannten Verbindungen kommt dem 3,7,11,15-Tetramethyl-1,2,3-trihydroxy-hexadecan (auch "Dihydro-2,3-dihydroxyphytol" oder "Phytantriol" genannt) die größte anwendungstechnische Bedeutung zu, es ist daher zur Durchführung der Erfindung besonders bevorzugt.

Durch den Zusatz von Polyvinylpyrrolidon kann die haarsplissverringernde Wirkung der erfindungsgemäßen Kombination aus Panthenol und Mono- oder Dissaccharid noch einmal verdoppelt werden. Hingegen zeigt der Zusatz von PVP zu d-Phantenol (ohne den Zusatz eines Mono- oder Disaccharids) keine Wirkung. Auch durch Zusatz von z.B. "Phytantriol" läßt sich eine weitere Verdopplung des bereits sehr guten Effektes der Kombination aus Panthenol und Mono- oder Disaccharid erreichen.

Die erfindungsgemäßen Haarbehandlungsmittel können in Form wässriger, wässrig-alkoholischer oder emulsionsförmiger Zubereitungen vorliegen. Im einfachsten Falle genügt eine Lösung der obligatorischen Komponenten gemäß Patentanspruch in Wasser oder wässrigem Alkohol als Träger. Es kommen jedoch alle bekannten Haarpflegemittel in ihrer typischen Zusammensetzung als Träger für die erfindungsgemäße Wirkstoffkombination infrage. Hierfür eignen sich nicht nur Haarpflegemittel, die nach der Anwendung auf dem Haar verbleiben, wie z.B. Haarwässer, Haarfestiger, Haarpomaden, sondern auch solche, die nach der Haarbehandlung vom Haar abgespült werden wie z.B. Shampoos, Spül- und Avivagelotionen, ja sogar Färbemittel und Dauerwell-Fixierlösungen.

Eine besonders bevorzugte Ausführungsform der Erfindung sind Haarnachspülmittel, wie sie z.B. nach dem Shamponieren des Haars angewendet werden, um dem Haar eine Avivage zu verleihen und die statische Aufladbarkeit des Haars zu verringern. Solche Haarnachspülmittel enthalten als antistatische Wirkstoffe wasserlösliche, oberflächenaktive quartäre Ammonium-, Pyridinium- oder Imidazoliniumverbindungen.

Eine weitere bevorzugte Ausführungsform sind daher erfindungsgemäße Haarbehandlungsmittel, die zusätzlich 0,1-5 Gew.-% einer solchen wasserlöslichen, oberflächenaktiven quartären Ammonium-, Pyridinium- oder Imidazoliniumverbindungen enthalten. Diese Verbindungen besitzen neben einer wasserlöslich machenden, quartären Ammonium-, Pyridinium- oder Imidazoliniumgruppe eine lipophile, lineare Alkyl- oder 2-Hydroxyalkylgruppe mit 8 bis 22 C-Atomen. Beispiele für solche Verbindungen sind z.B. Cetylpyridiniumchlorid, 2-Akyl(C₁₅)-1-(2ʹ-hydroxyethyl)-1-methyl-imidazolinium-methosulfat, oder 2-Undecyl-1-(2-hydroxyethyl)-1-methyl-imidazoliniumchlorid.

Unter den zahlreicen quartären Ammoniumverbindungen kommt den Verbindungen der Formel II 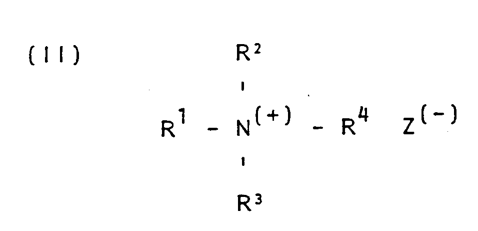
in der R¹ eine Alkyl- oder 2-Hydroxyalkylgruppe mit 8 bis 22 C-Atomen, eine Gruppe R⁵CONH(CH₂)ₓ-, worin R⁵ eine Alkylgruppe mit 7 bis 21 C-Atomen und x eine Zahl von 2 bis 4 ist, R² und R³ eine Alkylgruppe mit 1 bis 4 C-Atomen oder eine Gruppe der Formel -(CₚH₂ₚO)_{y}-H ist, worin p eine Zahl von 2 bis 4 und y eine Zahl von 1 bis 10 ist, und R⁴ eine Benzylgruppe ist oder eine der für R² und R³ angegebenen Bedeutung hat und Z⁻ ein Chlorid-, Bromid-, Hydrogensulfat, Hydrogenphosphat, Methoxysulfat oder Ethoxysulfat-Anion ist, eine besondere Bedeutung zu.

Besonders geeignete quartäre Ammoniumverbindungen dieser Art sind z.B. Cetyl-trimethylammoniumchlorid, 2-Hydroxycetyl-2-hydroxyethyl-dimethyl-ammoniumchlorid, Lauryltrimethylammoniumchlorid oder Behenyl-trimethyl-ammoniumchlorid.

Erfindungsgemäße Haarnachspülmittel liegen bevorzugt in der Form einer wässrigen Dispersion vor und enthalten
1 - 5 Gew.-% Cetyl- oder Stearylalkohol
0,1 - 5 Gew.-% einer wasserlöslichen, oberflächenaktiven, quartären Ammonium-, Pyridinium- oder Imidazoliniumverbindung und
0,1 - 1 Gew.-% eines wasserlöslichen Polysaccharid-Ethers.

Als wasserlösliche Polysaccharid-Ether kommen z.B. Methylcellulose, Ethylcellulose, Hydroxyethylcellulose, Hydroxypropylcellulose, Methyl-hydroxypropylcellulose sowie entsprechend durch Methyl, Hydroxyethyl- und/oder Hydroxypropylgruppen substituierte Stärke oder Guar-Derivate infrage.

Die haarregenerierende Wirkung, das heißt, die haarsplissverringernde Wirksamkeit der erfindungsgemäßen Haarbehandlungsmittel, kann durch Auswahl der Art und Menge der Komponenten erheblich gesteigert werden. So wird z.B. eine besonders starke Wirksamkeit erzielt, wenn diese Mittel
0,2 - 5 Gew.-% D(+)Panthenol
0,5 - 5 Gew.-% Glucose und
0,5 - 3 Gew.-% Polyvinylpyrrolidon enthalten.

Eine vergleichbar gute Wirkung wird erhalten, wenn die Mittel
0,2 - 5 Gew.-% D(+)Panthenol
0,5 - 5 Gew.-% Glucose und
0,1 - 1 Gew.-% 3,7,11,15-Tetramethyl-1,2,3-trihydroxy-hexadecan enthalten.

Neben Haarnachspülmitteln können auch Haarwaschmittel (Shampoos) als Träger für die erfindungsgemäße haarregenerierende Wirkstoffkombination dienen. Diese enthalten schaumstarke anionische Tenside, bevorzugt Sulfat- oder Sulfonat-tenside. Diese Tenside enthalten eine lipophile, bevorzugt geradkettige Alkylkette mit 10-18 C-Atomen und daran eine wasserlöslichmachende Sulfat- oder Sulfonatgruppe. Sie liegen als wasserlösliche Alkali-, Magnesium-, Ammonium- oder Mono-, Di- oder Trialkanolammoniumsalze (mit 2- oder 3 C-Atomen in der Alkanolgruppe) vor. Beispiele für geeignete Aniontenside sind Fettalkohol-(C₁₂-C₁₈)-sulfate, Fettalkohol(C₁₂-C₁₈)-polyglycolethersulfate mit 1-10 Glycolethergruppen, Fettsäure-(C₁₂-C₁₈)-monoethanolamid-polyglycolethersulfate mit 1-6 Glycolethergruppen, Sulfobernsteinsäure-mono-und/oder-diester von C₈-C₁₈-Fettalkoholen oder von C₈-C₁₈-Fettalkoholpolyglycolethern mit 1-10 Glycolethergruppen, Alpha-Sulfofettsäure(C₁₂-C₁₈)methylester, sekundäre Alkan(C₁₂-C₁₈)-sulfonate, Alpha-olefinsulfonate, und Alkyl-(C₈-C₁₈)-glycerylsulfate.

Weitere Tenside, die in den erfindungsgemäßen Zusammensetzungen zusätzlich enthalten sein können, sind die ampholytischen und zwitterionischen und die nichtionogenen Tenside. Unter ampholytischen Tensiden werden solche oberflächenaktiven Verbindungen verstanden, die außer einer C₈-C₁₈-Alkylgruppe eine freie Aminogruppe und eine -COOH- oder -SO₃H-Gruppe im Molekül tragen und zur Ausbildung innerer Salze befähigt sind. Zwitterionische Tenside tragen außer einer C₈-C₁₈-Alkylgruppe in ihrem Molekül eine quartäre Ammoniumgruppe und eine -COO⁽⁻⁾- oder -SO₃⁽⁻⁾-Gruppe. Beispiele für ampholytische Tenside sind z.B. die 2-Alkyl-(C₈-C₁₈)-aminopropionsäure oder Alkyl-(C₈-C₁₈)-aminoessigsäure. Beispiele für zwitterionische Tenside sind N-Alkyl-(C₈-C₁₈)-dimethyl-ammonio-glycinat, N-Acyl-(C₈-C₁₈)-aminopropyl-dimethyl-ammonio-glycinat, 2-Alkyl-(C₈-C₁₈)-3-carboxymethyl-3-hydroxyethyl- imidazolin. Nichtionische Tenside sind z.B. die Anlagerungsprodukte von 1 bis 30 Mol Ethylenoxid oder Propylenoxid an Fettalkohole mit 8 bis 22 C-Atomen, an C₁₂-C₁₈-Fettsäuren, an C₁₂-C₁₈-Fettamine, an Alkylphenole mit 8 bis 15 C-Atomen in der Alkylgruppe, an Fettsäure-(C₁₂-C₁₈)-monoethanolamide, an Fettsäure (C₁₂-C₁₈)-mono- und -diglyceride und an Fettsäure (C₁₂-C₁₈)sorbitan-partialester. Weitere geeignete nichtionische Tenside sind Zucker-Fettsäureester, Alkylglucoside und Alkyloligoglucoside mit 8 bis 18 C-Atomen in der Alkylgruppe und Methylglucosid-Fettsäurepartialester sowie deren Ethylenoxidanlagerungsprodukte und die Glycerin-oxethylat-Fettsäureester. Schließlich gehören auch die tertiären Aminoxid-Tenside zu den geeigneten nichtionischen Tensiden.

Die erfindungsgemäßen Haarbehandlungsmittel können auch noch weitere Komponenten enthalten, die für die jeweilige Anwendungsform üblich sind, z.B.
- Fettsäure(C₁₂-C₁₈)alkanolamide als Verdickungsmittel und Schaumverstärker
- Salze, z.B. NaCl, Na₂SO₄, MgCl₂, MgSO₄ zur Viskositätseinstellung und zur Verbesserung der Strukturant-Eigenschaften
- Wasserlösliche Polymere mit kationischen Gruppen, z.B. Celluloseether mit quartären Ammoniumgruppen als avivierende Komponente,
- Wasserlösliche anionische Polymere, z.B. wasserlösliche Polymere mit Carboxylat-Gruppen zur Verbesserung der haarfestigenden Eigenschaften
- Kosmetische Wachs-, Fett- und Ölkomponenten, Silikonöle (Polydimethylsiloxane)
- Niedere Alkohole und Glycole wie Ethanol, Isopropanol, Propylenglycol als Lösungsvermittler,
- Sebostatische und Antischuppen-Wirkstoffe
- Farbstoffe
- Duftstoffe
- Konservierungsstoffe.

Wie weiter oben ausgeführt, kann die haarregenerierende Wirkstoffkombination auch in Haarfärbemittel oder in Dauerwellfixiermittel eingebracht werden. Diese Zubereitungen können alle für solche Präparate übliche Bestandteile enthalten, etwaige Unverträglichkeiten sind nicht beobachtet worden.

Die folgenden Beispiele sollen den Gegenstand der Erfindung näher erläutern.

### Beispiele

### Prüfung der haarregenerierenden Wirkung

Es wurden Haarspülmittel gemäß den in Tabelle I angegebenen Zusammensetzungen hergestellt. Bei der Herstellung der wässrigen Dispersionen gemäß Beispiel 1-6 wurde wie folgt gearbeitet:
Cetyl-/Stearylalkohol (50:50) und Glycerinmono/distearat/palmitat wurden gemeinsam auf 80 °C erhitzt und im geschmolzenen Zustand vermischt. Die Lösung des Cetyltrimethylammoniumchlorids in der Wassermenge wurde auf 80 °C erwärmt und unter Rühren zur Schmelze gegeben. Die sich bildende Emulsion wurde ohne Wärmezufuhr weiter gerührt. Nach Abkühlung auf ca. 40 °C wurde das Panthenol und ggf. die Glucose, das Polyvinylpyrrolidon und Phytantriol zugegeben. Schließlich wurden 200 g einer Lösung von 3 Gew.-% Methyl-hydroxypropylcellulose in Wasser zugesetzt und die Dispersion weiter bis zur Abkühlung auf 25 °C langsam gerührt.

Bei der Herstellung der Zubereitung gemäß Beispiel 7 wurden Cetyltrimethylammoniumchlorid, d-Panthenol, Glucose und Polyvinylpyrrolidon bei 30 °C in dem Wasser aufgelöst.

Die Prüfung der haarregenerierenden Wirkung erfolgte anhand einer Probe von 100, durch mechanische und elektrostatische Vorbehandlung gesplissten Haaren. Diese wurde 10 Minuten lang mit den unverdünnten Zubereitungen gemäß Beispiel 1-7 behandelt. Dann wurden die Haare mit Leitungswasser ausgespült, getrocknet und gekämmt. Sodann wurde die visuell noch erkennbare Splissrate durch Auszählen der gesplissten Haare bestimmt.

Es zeigte sich in allen Fällen eine Regenerierung der Haare, das heißt, die gespaltenen Haarspitzen waren zum Teil wieder repariert.

Mit der Zusammensetzung gemäß Beispiel 2 wurde zusätzlich eine Prüfung der beschriebenen Art ohne Ausspülen des Haarbehandlungsmittels durchgeführt. In diesem Fall wurde das Behandlungsmittel lediglich mechanisch (durch Abquetschen) aus dem Haar entfernt. Nach dem Trocknen und Kämmen betrug die Splissrate nur noch 10 %. Daran erkennt man, daß die Vorteile der erfindungsgemäßen Haarbehandlungsmittel insbesondere bei abspülbaren Präparaten zum tragen kommen.

### Ergebnis:

Die Beispiele 1-3 zeigen die Wirkung der (nicht erfindungsgemäßen) Zusammensetzungen mit Panthenol (Beispiel 1 und 2) bzw. mit Panthenol und PVP (Beispiel 3). In Beispiel 4 wird die synergistisch gesteigerte Wirkung der Kombinaton aus d-Panthenol und Glucose sichtbar. Die Beispiele 5 und 6 zeigen, daß durch PVP oder Phytantriol eine weitere Wirkungssteigerung erreicht wird. Beispiel 7 zeigt, daß die Wirkung auch durch klare, wässrige Zubereitungen erzielt wird.

Die Ergebnisse sind in Tabelle I zusammengefaßt.

**Tabelle I**

| | 1 | 2 | 3 | 4 | 5 | 6 | 7 |
|---|---|---|---|---|---|---|---|
| Cetyl-Stearylalkohol | 4 | 4 | 4 | 4 | 4 | 4 | - |
| GMS ¹⁾ | 3 | 3 | 3 | 3 | 3 | 3 | - |
| CTAC ²⁾ | 2 | 2 | 2 | 2 | 2 | 2 | 1 |
| MHPC (3%ige Losung) ³⁾ | 20 | 20 | 20 | 20 | 20 | 20 | - |
| Wasser | 70 | 66 | 64 | 61 | 59 | 63,8 | 90 |
| d-Panthenol | 1 | 5 | 5 | 5 | 5 | 5 | 5 |
| Glucose-Monohydrat | - | - | - | 5 | 5 | 2 | 2 |
| PVP ⁴⁾ | - | - | 2 | - | 2 | - | 2 |
| Phytantriol | - | - | - | - | - | 0,2 | - |
| Spliss Rate | 50 | 35 | 50 | 20 | 10 | 10 | 20 |

| | | | | | | | |
|---|---|---|---|---|---|---|---|
| 1) GMS: Glycerin-Mono/Di-Stearat-/Palmitat | | | | | | | |
| 2) CTAC: Cetyl-trimetyhl-ammonium-chlorid | | | | | | | |
| 3) MHPC: Methyl-hydroxypropylcellulose/mittleres Molgewicht ca. 3 000 (3 %ige Lösung in Wasser) | | | | | | | |
| 4) PVP: Polyvinylpyrrolidon (mittleres MG: ca. 40 000 (Luviskol^{(R)} K 30) | | | | | | | |

## Patentansprüche

1. Haarbehandlungsmittel in Form wässriger, wässrig-alkoholischer oder emulsionsförmiger Zubereitungen zur Pflege strapazierter Haare, dadurch gekennzeichnet, daß sie
0,1 - 8 Gew.-% Panthenol und
0,1 - 8 Gew.-% eines Mono- oder Disaccharids enthalten.

2. Haarbehandlungsmittel nach Anspruch 1, dadurch gekennzeichnet, daß zusätzlich
0,1 - 4 Gew.-% Polyvinylpyrrolidon und/oder
0,01 - 1 Gew.-% eines Triols der allgemeinen Formel I 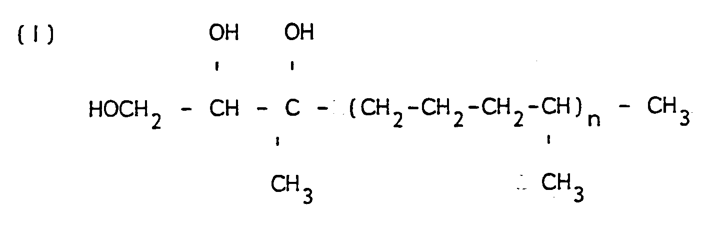 in der n = 1-3 ist, enthält.

3. Haarbehandlungsmittel nach Anspruch 1 oder 2, dadurch gekennzeichnet, daß sie zusätzlich
0,1 - 5 Gew.-% einer wasserlöslichen, oberflächenaktiven quartären Ammonium-, Pyridinium- oder lmidazoliniumverbindung enthalten.

4. Haarbehandlungsmittel nach Anspruch 1 bis 3, dadurch gekennzeichnet, daß sie in Form einer wässrigen Dispersion mit einem Gehalt von
1 - 5 Gew.-% Cetyl- und/oder Stearylalkohol
0,1 - 5 Gew.-% einer wasserlöslichen, oberflächenaktiven quartären Ammonium-, Pyridinium- oder Imidazoliniumverbindung und
0,1 - 1 Gew.-% eines wasserlöslichen Polysaccharid-Ethers vorliegen.

5. Haarbehandlungsmittel nach Anspruch 1 bis 4, dadurch gekennzeichnet, daß sie
0,2 - 5 Gew.-% d-Panthenol
0,5 - 5 Gew.-% Glucose
0,5 - 3 Gew.-% Polyvinylpyrrolidon enthalten.

6. Haarbehandlungsmittel nach Anspruch 1 bis 4, dadurch gekennzeichnet, daß sie
0,2 - 5 Gew.-% d-Panthenol
0,5 - 5 Gew.-% Glucose und
0,1 - 1 Gew.-% 3,7,11,15-Tetramethyl-1,2,3-trihydroxy-hexadecan enthalten.

## Claims

1. Hair-treatment preparations in the form of aqueous, aqueous-alcoholic or emulsion-like preparations for the care of mismanaged hair, characterized in that they contain
0.1 to 8% by weight panthenol and
0.1 to 8% by weight of a mono- or disaccharide.

2. Hair-treatment preparations as claimed in claim 1, characterized in that they additionally contain
0.1 to 4% by weight of a polyvinyl pyrrolidone and/or
0.01 to 1% by weight of a triol corresponding to the following formula 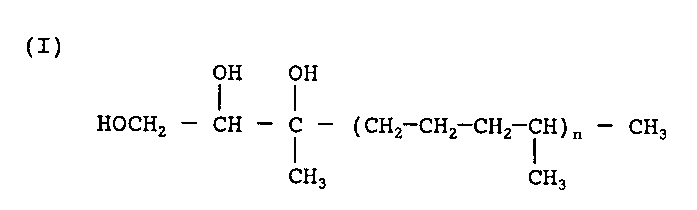 in which n = 1 - 3.

3. Hair-treatment preparations as claimed in claim 1 or 2, characterized in that they additionally contain
0.1 to 5% by weight of a water-soluble, surface-active quaternary ammonium, pyridinium or imidazolinium compound.

4. Hair-treatment preparations as claimed in claims 1 to 3, characterized in that they are in the form of an aqueous dispersion containing
1 to 5% by weight cetyl and/or stearyl alcohol,
0.1 to 5% by weight of a water-soluble, surface-active quaternary ammonium, pyridinium or imidazolinium compound and
0.1 to 1% by weight of a water-soluble saccharide ether.

5. Hair-treatment preparations as claimed in claims 1 to 4, characterized in that they contain
0.2 to 5% by weight d-panthenol,
0.5 to 5% by weight glucose,
0.5 to 3% by weight polyvinyl pyrrolidone.

6. Hair-treatment preparations as claimed in claims 1 to 4, characterized in that they contain
0.2 to 5% by weight d-panthenol
0.5 to 5% by weight glucose and
0.1 to 1% by weight 3,7,11,15-tetramethyl-1,2,3-trihydroxy-hexadecane.

## Revendications

1. Produits pour le traitement des cheveux sous la forme de préparations aqueuses, aqueuses-alcooliques ou d'émulsions pour soigner les cheveux abîmés, qui sont caractérisés en ce qu'il renferment
0,1 à 8 % en poids de panthénol et
0,1 à 8 % en poids d'un mono- ou d'un disaccharide.

2. Produits pour le traitement des cheveux selon la revendication 1, caractérisés en ce qu'ils contiennent en plus:
0,1 à 4% en poids d'une polyvinylpyrrolidone et/ou
0,01 à 1 % en poids d'un triol de la formule générale I 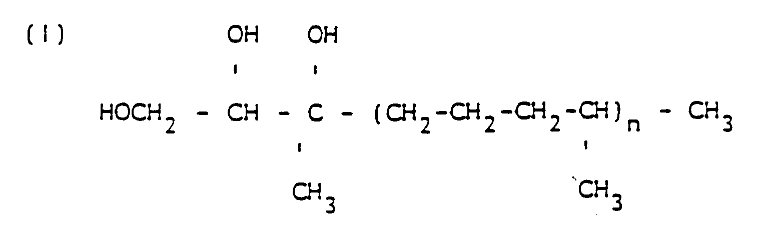 dans laquelle n = 1 à 3.

3. Produits pour le traitement des cheveux selon la revendication 1 ou 2, caractérisés en ce qu'ils contiennent en plus:
0,1 à 5 % en poids d'un composé d'ammonium quaternaire, de pyridinium ou d'imidazolinium tensioactif et soluble dans l'eau.

4. Produits pour le traitement des cheveux selon les revendications 1 à 3, caractérisés en ce qu'ils sont sous la forme d' une dispersion aqueuse renfermant
1 à 5 % en poids d'alcool cétylique et/ou stéarylique
0,1 à 5 % en poids d'un composé d'ammonium quaternaire, de pyridinium ou d'imidazolinium tensioactif et soluble dans l'eau et
0,1 à 1 % en poids d'un éther polysaccharidique soluble dans l'eau.

5. Produits pour le traitement des cheveux selon les revendications 1 à 4, caractérisés en ce qu'ils contiennent
0,2 à 5 % en poids de d-panthénol
0,5 à 5 % en poids de glucose
0,5 à 3 % en poids de polyvinylpyrrolidone.

6. Produits pour le traitement des cheveux selon les revendications 1 à 4, caractérisés en ce qu'ils contiennent
0,2 à 5 % en poids de d-panthénol
0,5 à 5 % en poids de glucose
0,1 à 1 % en poids de 3,7,11,15-tétraméthyl-1,2,3-trihydroxy-hexadécane.
